(19) Europäisches Patentamt
European Patent Office
Office européen des brevets

(11) **EP 2 760 329 B1**

(12) **FASCICULE DE BREVET EUROPEEN**

(45) Date de publication et mention de la délivrance du brevet:
**11.01.2023   Bulletin 2023/02**

(21) Numéro de dépôt: **12784465.2**

(22) Date de dépôt: **27.09.2012**

(51) Classification Internationale des Brevets (IPC):
**A61B 3/11** *(2006.01)*      **G02C 13/00** *(2006.01)*
**A61B 3/113** *(2006.01)*

(52) Classification Coopérative des Brevets (CPC):
**G02C 13/005; G02C 13/003;** A61B 3/111

(86) Numéro de dépôt international:
**PCT/EP2012/069044**

(87) Numéro de publication internationale:
**WO 2013/045531 (04.04.2013 Gazette 2013/14)**

(54) **PROCEDE DE DETERMINATION DE MESURES OCULAIRES ET OPTIQUES**

VERFAHREN ZUR BESTIMMUNG OKULARER UND OPTISCHER MESSUNGEN

METHOD FOR DETERMINING OCULAR AND OPTICAL MEASUREMENTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **29.09.2011   FR 1102952**
              **18.11.2011   FR 1160547**

(43) Date de publication de la demande:
**06.08.2014   Bulletin 2014/32**

(73) Titulaire: **Fittingbox**
**31670 Labège (FR)**

(72) Inventeurs:
• **LE GALLOU, Sylvain**
**F-31450 Baziege (FR)**
• **CHOUKROUN, Ariel**
**F-31000 Toulouse (FR)**

(74) Mandataire: **Ipside**
**6, Impasse Michel Labrousse**
**31100 Toulouse (FR)**

(56) Documents cités:
FR-A1- 2 914 173      FR-A1- 2 915 290
US-A1- 2007 279 590   US-A1- 2009 109 400

## Description

La présente invention concerne le domaine des mesures oculaires et optiques, et plus particulièrement un procédé de détermination de mesures oculaires et optiques.

## Préambule et art antérieur

Les mesures oculaires et optiques ont pour objectif le montage des verres sur les montures portées. Le montage des verres doit assurer une correction idéale ainsi qu'un confort de vision pour les activités quotidiennes usuelles (conduire, lire, etc.) qui représentent chacune des conditions d'accommodation et de position de l'œil par rapport au verre différentes. Plusieurs mesures sont nécessaires à la fabrication et au montage de verre.

Actuellement les grandeurs utilisées sont celles des activités en vision de loin, qui correspondent à un regard à l'infini (> 2m), et en vision de près (>30cm et <2m) et les mesures suivantes :

- mesure pour la vision des corrections simples (ou SV pour Single Vision la selon la terminologie anglaise) pour des verres unifocaux :
  ◦ Distance Pupillaire (PD) : la distance pupillaire est la distance entre les centres des pupilles lorsque le sujet regarde à l'infini. Cette mesure concerne le système des yeux uniquement.
- mesures pour la vision des corrections fortes et/ou progressives (PV pour Progressive Vision selon la terminologie anglaise) pour des verres varifocaux ou progressifs: ces mesures ont pour objectif de permettre le montage précis du verre en considérant la monture portée. Elles concernent donc l'ajustement de la vision pour une monture portée et ajustée pour le visage, en considérant un regard à l'infini et parfaitement aligné avec la direction du visage.

  ◦ Distance monoPupillaire (MonoPD) : distance entre la projection de la pupille dans le plan du verre et le centre de la monture. Cette grandeur mesure le décentrement horizontal à appliquer lors du taillage du verre.
  ◦ Hauteurs (Segment Heights) : distance entre la projection de la pupille dans le plan du verre et le bas de la monture (intérieur présumé de la dragée interne). Cette grandeur mesure le décentrement vertical à appliquer lors du taillage du verre.

Ces mesures correspondent à une visée de l'œil à l'infini. Les visions de près peuvent être mesurées pour des distances considérées fixes (40cm), et elles sont en général déduites à partir de tables.

En pratique, l'œil est mis en situation de visée et les outils permettent de mesurer l'endroit où le rayon visé par l'œil intersecte le verre. Cela permet de prendre en compte implicitement les paramètres de visée du sujet : strabisme léger, capacité de convergence, d'accommodation, et oeil directeur en fonction de la position et de l'orientation par rapport au point visé en vision binoculaire.

En fonction de la correction, les verres sont taillés pour gérer les décalages de visée dus au verre lui-même. Pour cela, la distance verre-œil et l'angle verre oeil en configuration de regard infini pour une tenue de tête neutre sont pris en compte. On considère là aussi des abacs, avec une distance moyenne verre oeil de 13mm et un angle moyen, appelé angle pentoscopique, à 10 degrés.

Cependant, si ces défauts sont significatifs, la mesure de la vision de près devient nécessaire, tout en prenant en compte la situation de visée : lecture, travail sur ordinateur, ... autant de situations qui ont des hauteurs de visée différentes par rapport au visage et qui varient avec les habitudes de la personne.

Actuellement, la fabrication de verres de haute technologie permet d'adapter la vue aux différents angles et distances de visée. Chaque oeil étant différent, et la mesure étant définie pour être effectuée dans le plan du verre, il est donc utile d'avoir autant de mesures que de besoins de visée différents. En pratique, l'opticien n'a pas le temps de les effectuer avec des outils classiques.

Il existe ainsi différentes sources d'erreurs qui se combinent et s'ajoutent lors d'un montage de verres sur une paire de lunettes :

- L'erreur de mesure sur les formes de la monture : en général, l'opticien met la paire dans une machine qui palpe la dragée interne de la monture : erreur +-0.2mm. Pour les montures non cerclées, on mesure la taille du verre de démonstration ou l'opticien choisit la forme avec son client.
- L'erreur de taillage du verre: la norme NF EN ISO 21987 donne des valeurs d'erreurs maximales. Des guides de bonnes pratiques de réalisation pour les opticiens indiquent en pratique une erreur maximum de 1.5mm pour l'erreur entre centrage horizontal demandé et réalisé, et 1mm maximum entre centrage vertical demandé et réalisé pour les verres progressifs.
- L'erreur de mesure interpupillaire : erreur commise sur le PD, monoPD et hauteur. Pas de recommandation officielle, mais des valeurs pratiques obtenues de l'ordre du demi- millimètre pour le PD, et du millimètre par oeil pour les monoPD et hauteurs.

Il existe des tables d'erreurs maximum conseillées sur le montage final en fonction du degré de correction et du type de verres. Par soustraction, on en déduit que les qualités de mesure interpupillaires actuelles sont suffisantes. Mais pour cela il est nécessaire que les mesures soient réalisées correctement. Des procédés standards de détermination des mesures oculaires et optiques sont décrits par exemple dans les documents FR2914173 et FR2915290 qui décrivent l'utilisation d'une mire prédéterminée, non disponible à un utilisateur particulier mais seulement à un opérateur spécialisé, la mire étant définie ou attachée sur une paire de lunettes particulière, et les mesures étant analysées une par une et non de façon globale par modélisation du système de l'oeil.

**Exposé de l'invention**

La présente invention a donc pour objet de palier un ou plusieurs des inconvénients de l'art antérieur en proposant un procédé de détermination des mesures oculaires et optiques pour la fabrication et le montage de verre de lunettes correcteurs permettant des mesures plus précises.

Pour cela la présente invention propose un procédé de détermination des mesures oculaires et optiques pour la fabrication et le montage de verre de lunettes correcteurs pour un utilisateur, à l'aide d'une caméra, utilisant un protocole de reconstruction du système des yeux de l'utilisateur en trois dimensions par modélisation du système de l'œil.

L'invention permet ainsi de définir des verres adaptés à l'œil de l'utilisateur de manière très simple à l'aide d'une camera. L'ensemble des mesures est effectuées directement en même temps.

Selon un mode de réalisation de l'invention, le protocole comprend au moins une étape consistant à effectuer les mesures oculaires et optiques pour différents points de visée et différentes orientations de visage de l'utilisateur.

Selon un mode de réalisation de l'invention, les points de visée sont au nombre de trois et les orientations de visage de l'utilisateur sont au nombre de trois

Selon un mode de réalisation de l'invention, la modélisation est réalisée à partir des grandeurs réelles de l'œil qui sont : la taille de l'iris, la taille des globes oculaires, l'orientation des globes oculaires dans leur cavité, et la pose de l'ensemble des deux yeux dans un repère de la caméra.

Selon un mode de réalisation de l'invention, le procédé comprend une étape de mesure de la distance interpupillaire (PD3D) entre les deux yeux reconstruits en trois dimensions.

Selon un mode de réalisation de l'invention, le procédé comprend une étape de mesure de la distance monopupillaire (monoPD), des hauteurs, de l'angle pentoscopique, directement dans le système trois dimensions de l'œil reconstruit.

Selon un mode de réalisation de l'invention, le procédé comprend une étape d'alignement des indices images.

Selon un mode de réalisation de l'invention, le procédé utilise des objets mire solidaires ou non solidaires du visage de l'utilisateur.

Selon un mode de réalisation de l'invention, la mire à la forme d'une carte rectangulaire.

Selon un mode de réalisation de l'invention, la mire est contenue dans un écran de visualisation.

Selon un mode de réalisation de l'invention, la détermination de la taille métrique de l'écran se fait grâce à un objet plan

Selon un mode de réalisation de l'invention, la mire est utilisée au début du protocole.

Selon un mode de réalisation de l'invention, le visage de l'utilisateur sert d'objet mire pour le système des yeux.

Selon un mode de réalisation de l'invention, le procédé comprend une étape de mesures PV avec un essayage virtuel de lunettes trois dimensions.

Selon un mode de réalisation de l'invention, le procédé comprend une étape de calibrage et de définition métrique de la caméra.

Selon un mode de réalisation de l'invention, le procédé comprend une étape de calibrage géométrique de l'écran.

Selon un mode de réalisation de l'invention, leprocédé comporte une étape d'évaluation de la stabilité et des performances ce qui permet de détecter les défauts de réalisation et de les corriger.

Selon un mode de réalisation de l'invention, le procédé est interactif et en temps réel.

Selon un mode de réalisation de l'invention, le procédéest automatique.

Selon un mode de réalisation de l'invention, le procédé comprend une étape d'analyse du comportement oculaire.

Selon un mode de réalisation de l'invention, le procédé a une architecture Client-Serveur.

Selon un mode de réalisation de l'invention, le procédéfonctionne avec deux caméras reliées par une contrainte rigide connue ou inconnue.

Selon un mode de réalisation de l'invention, le procédé fonctionne avec au moins un dispositif donnant la profondeur.

**Présentation des figures**

D'autres caractéristiques et avantages de l'invention seront mieux compris et apparaîtront plus clairement à la lecture de la description faite, ci-après, en se référant aux figures annexées et données à titre d'exemple:

- la figure 1 est une représentation schématique d'une vue dans l'espace d'un système de reconstruction 3D,

- la figure 2 est une représentation schématique d'une autre vue du système de reconstruction 3D,

- la figure 3 est une représentation schématique d'un protocole de reconstruction,

- la figure 4 est une représentation schématique d'un autre protocole de reconstruction,

- la figure 5 représente l'étape de mesure de la monoPD,

- la figure 6 représente deux variantes de l'étape de mesure de la hauteur.

- la figure 7 représente des photographies du protocole de mesure du PD avec une mire carte de crédit. a) Regard point central ; b) Regard d'un point sur le coté de l'écran,

- la figure 8 représente la carte a) et le CD b) utilisés lors du calibrage de l'écran.

**Description détaillée d'un mode de réalisation de l'invention**

Le contexte général de l'invention est celui de la détermination, au moyen d'une caméra vidéo de définition standard, de mesures oculaires et optiques permettant la fabrication et le montage précis de verres correcteurs sur une monture de lunettes. Deux contextes de mise en oeuvre sont envisagés, le contexte magasin, qui correspond à l'installation d'un matériel spécifique chez un opticien et le contexte en ligne, qui correspond à l'utilisation d'un matériel standard par un utilisateur quelconque relié à Internet.

On distingue également deux ensembles de mesures à déterminer, en fonction du type de correction visée. La correction dite correction simple (SV) correspond à la fabrication et au montage de verres corrigeant les mêmes défauts de vision sur toute leur surface. La correction dite correction progressive (PV) correspond à la fabrication et au montage de verres corrigeant des types de défauts différents à différents points de leur surface (verres « progressifs »).

Ces quatre contextes ont en commun l'utilisation d'une seule caméra de définition quelconque et d'un environnement lumineux non spécifique.

Le système proposé par l'invention permet d'obtenir les mesures :

- de distance pupillaires (PD) : la **distance pupillaire** est la distance entre les centres des pupilles lorsque le sujet regarde à l'infini. Cette mesure concerne le système des yeux uniquement.
- de **distance monoPupillaire (MonoPD)** : distance entre la projection de la pupille dans le plan du verre et le centre de la monture. Cette grandeur mesure le décentrement horizontal à appliquer lors du taillage du verre.
- De hauteurs **(Segment Heights)** : distance entre la projection de la pupille dans le plan du verre et le bas de la monture (intérieur présumé de la dragée interne). Cette grandeur mesure le décentrement vertical à appliquer lors du taillage du verre.
- angle pentoscopique, et
- toutes les mesures nécessaires au montage de verres pouvant découler du port de la lunette et de la visée attendue,

avec une précision supérieure à celle des systèmes connus dans ce domaine, ainsi qu'une accessibilité maximale.

Pour un système de mesure oculaire la précision est définie par trois valeurs : la précision, la stabilité, et la répétabilité de l'outil. La précision finale en conditions réelles est la capacité du système à atteindre la précision lors de la mesure.

- La **précision** : c'est l'écart maximum par rapport à la valeur réelle pour une grande majorité des mesures. La précision qualifie le biais commis par l'instrument de mesure. C'est la mesure utile pour le taillage des verres par exemple, car il suffit de mesurer la forme et les distances réalisées par rapport aux données attendue. La précision finale en conditions réelles est la capacité du système à atteindre la précision lors de la mesure.
- La **stabilité** : c'est l'écart maximum autour de la valeur réelle pour les mesures hors de la plage de précision. La stabilité mesure l'influence des erreurs. Cette mesure correspond à la capacité de l'instrument à garantir la précision dans des situations différentes (personnes, environnement expérimental, etc.). Le système de mesure et son protocole associé doivent garantir que la précision est atteinte dans un certain pourcentage de cas, idéalement très élevé. Cette mesure exprime aussi la capacité de l'outil à éviter les erreurs grossières. En fonction du protocole de l'outil, on peut définir une nouvelle loi de précision pour la stabilité. On évalue alors la gravité et les implications de ce type d'erreurs en fonction de la grandeur observée.

- La **répétabilité** : c'est le nombre de mesures unitaires nécessaires pour garantir la précision. Il ne s'agit plus du maximum de l'erreur mais de l'erreur observée en moyenne en répétant un grand nombre de fois la mesure (convergence asymptotique). En pratique, cela donne une indication sur la fiabilité de n'effectuer qu'une fois la mesure avec l'instrument, ou de devoir la vérifier plusieurs fois.

Afin d'éviter de devoir redéfinir la distance pupillaire PD pour chaque angle de visée, on redéfinie cette grandeur indépendamment de ce paramètre, et on l'appelle PD3D. PD3D est la distance entre les centres de rotation des deux yeux. Cette définition s'appuie sur le fait que le point de formation de l'image (centre optique) de chaque oeil est confondu avec son centre de rotation ([Optometry product Guide, Zeiss], [Ophthalmic lenses and dispensing, Butterworth-Heinemann Optician, 1999 ISBN0750641584]). D'autres définitions existent, en fonction de là où la zone de formation de l'image est considérée, mais la plupart reviennent à mesurer des points proches de 0,1mm.Cette mesure correspond à la mesure de la distance pupillaire PD à l'infini, en l'absence de strabisme. Cependant, l'intérêt de cette mesure est qu'elle permet, lors de l'observation d'un sujet regardant à l'infini puis près, de mesurer les distances pupillaires PD à l'angle désiré.

Les mesures monoPD, Hauteurs sont redéfinies avec pour référence le point 3D proposé. Le taillage du verre varifocal sera alors calculé en fonction de la position et orientation de la monture, et des activités de visée les plus fréquentes du sujet. Un verre taillé pour des mesures à l'infini correspondra aux mesures utilisées actuellement.

Le système est constitué d'une unité centrale, d'une webcam quelconque reliée à cette unité, d'un écran classique de visualisation, et d'une mire. Pour certaines mesures et selon certaines configurations, la monture peut être utilisée pour certaines prises de mesure. Le support matériel peut prendre la forme d'un ordinateur fixe ou portable, mais aussi de tablettes ou plateformes mobiles (téléphones). L'unité de calcul peut être répartie sur plusieurs supports et certains calculs déportés si la plateforme d'utilisation est reliée à internet.

Le logiciel permettant l'acquisition et les calculs peut prendre la forme d'une application et/ou d'un site internet pour navigateur. Enfin, la mire peut prendre différentes formes et tailles. La mire proposée est un objet standard qui permet à chacun d'accéder au service de prise de mesures sans matériel particulier partout dans le monde. Les caractéristiques de la mire font que le système proposé par l'invention est non invasif, ou bien moins que les systèmes actuels. D'autre part, cette mire n'a pas besoin d'être attaché à la monture de lunettes.

Enfin, le protocole d'utilisation est très simple à comprendre par tout utilisateur, ce qui facilite l'accessibilité de l'invention. Contrairement aux systèmes existants, la mesure se fait automatiquement sans qu'un opérateur qualifié externe ait à intervenir pour permettre au protocole d'aboutir, ce qui est le cas dans tous les systèmes existants, et présente le risque de mauvaise application du protocole et de mauvaise répétabilité du résultat.

L'invention permet d'utiliser tout matériel existant du marché, et de garantir la précision désirée pour des images acquises avec des webcams dans des conditions de prise de vue variables : lumière environnante, bruit de caméra, résolution, compression de l'image, changement de couleurs, etc. L'utilisation de matériel standard et répandu permet l'utilisation du procédé selon l'invention à domicile aussi bien qu'en magasin. Le protocole garantit des résultats précis y compris pour des images de faible résolution (dès 640 pixels x480 pixels).
La super-résolution atteinte dépend du principe d'alignement des indices image, et de son utilisation avec la résolution trois dimensions (3D) globale.

L'invention se différencie des systèmes actuels qui s'appuient sur le report de la mesure d'une longueur connue sur la mesure dans une image de face de la distance entre les deux yeux, ainsi que du report seul de mesure de la distance entre les pupilles en les considérant dans un plan orienté 3D connu. Ces deux méthodes étant imprécises, instables, et dépendant d'un très bon matériel de prise de vue et d'un regard à l'infini parfait, ce qui n'est jamais le cas en pratique.

Le modèle ici exposé reconstruit tous les phénomènes modélisés en 3D et s'abstrait des problèmes de qualité, résolution d'image et de taille de mire. On définit alors une distance interpupillaire entre les deux yeux 3D, définie dans le document de référence comme étant la distance entre les centres de rotation des globes oculaires.

Les mesures monoPD, hauteurs, angle pentoscopique, sont mesurées directement dans l'espace 3D reconstruit métriquement en considérant le système des yeux, du visage et des lunettes en 3D.

Le protocole associé à la méthode de résolution permet de trouver les mesures désirées quels que soient les défauts de vision, l'âge, la capacité de la personne à ouvrir les yeux, et son exécution est très facile à assimiler. La méthode de résolution repose sur la composition de différents ports de tête et directions de regard.

- Protocole privilégié : l'utilisateur tourne la tête en fixant les différents points présentés à chaque ligne par les lignes de visées. Les lignes de visées ne sont pas nécessairement exactement sur le point affiché à l'écran. Ici, 3 points de visée et plusieurs orientations de visage capturées lors du mouvement.

- Protocole alternatif : avec plusieurs points de visée (ici 7) et 3 configurations d'orientation de visage. Ce protocole peut être utilisée pour la modélisation alternative de résolution sans mire ni point 3D du visage connu.

Il est proposé plusieurs types de mire, solidaires au visage, mais de position non nécessairement constante sur la surface du visage au cours de l'expérience. Ces objets peuvent être de petite taille et peuvent être plans, ce qui constitue une nouveauté et une avancée scientifique. Ainsi, un compact-disc (CD), une carte format carte de crédit rectangle sont des mires fonctionnelles pour le système proposé. Contrairement à tous les procédés qui utilisent une carte, ces derniers établissent un report de mesure dans l'espace qui donne lieu à des erreurs conséquentes.

La mire peut être également n'importe quelle monture dont on a effectué une reconstruction 3D. Ces dernières mires ont l'avantage de donner les mesures PV dans le même protocole d'acquisition.

Enfin, il est proposé un protocole et une résolution tels que la mire n'est plus sur le visage mais contenue dans l'écran de visualisation.

Il est proposé une variante de solution dans laquelle la mire est utilisée au début, puis le protocole initial décrit peut s'effectuer sans objet sur le visage. Dans un premier protocole, le visage est reconstruit en 3D et la métrique est apportée par la mire. Dans un second temps, le protocole principal est effectué, et le visage est devenu l'objet mire pour le système des yeux.

Dans le cas ou l'on connaît des points 3D du visage ainsi que leurs relations métriques, alors ce visage peut être utilisé comme mire pour la résolution du PD et les mesures PV.

Une reconstruction métrique de la scène 3D vue par une caméra n'est possible que si cette dernière est calibrée. Notre procédé n'impose pas une caméra particulière, il s'adapte à chaque caméra via une étape calibrage utilisant une des mires proposées.

Une fois la métrique 3D du système des yeux et de la mire obtenue, il est proposé de prendre les mesures PV en plaçant virtuellement le modèle 3D de la paire de lunettes choisie. La représentation 3D de la paire virtuelle permet de simuler son placement sur le visage de l'utilisateur, et de prendre les mesures PV 3D une fois le placement effectué. Ce placement peut être automatique ou manuel.

Le système est stable et propose une mesure de la confiance dans le résultat atteint, ce qui permet de détecter les défauts de réalisation et de les corriger.

Le procédé étant interactif et en temps réel, le retour sur la réalisation est immédiat et le guidage est facilité.

## Détermination de la distance pupillaire (PD) en contexte mono-caméra

Le système selon l'invention propose les caractéristiques d'un système de mesure fiable qui garantit les mesures de précision, stabilité et répétabilité définies plus haut.

Le système permet de mesurer l'ensemble des grandeurs associées à des regards et orientations de tête définis. Le système peut ainsi permettre autant de mesures adaptées à la vision du sujet, la précision désirée sur les verres et les activités de vision considérées. A cet égard le PD3D est considéré comme grandeur primordiale, car elle mesure les contraintes mécaniques du système des yeux en s'affranchissant des problèmes de vision et des conditions d'ordre contextuel.

La qualité de la mesure est assurée par la possibilité d'étalonner le système lorsque cela est nécessaire ou pour contrôle.

Le système est automatisé. En effet, il est important qu'il soit le plus automatique possible afin d'éviter toute erreur humaine, qui ajoute à la dépendance de facteurs extérieurs : fatigue, manque de formation ou d'expérience, mauvaise interprétation (de l'image par exemple), etc. Lorsqu'une intervention manuelle est nécessaire, le système doit pouvoir la contrôler et la qualifier.

L'automatisation du système concerne aussi la capacité à rejouer l'expérience pour effectuer un nombre de fois suffisant la mesure et assurer la répétabilité de chaque instance du protocole.

Le système propose une fonctionnalité d'évaluation de son état, par des actions automatiques ou des moyens de contrôle simples à mettre en oeuvre. L'évaluation et le contrôle des opérations manuelles éventuelles permettent d'assurer une intégration de l'humain mesurable et contrôlée.

Un mécanisme d'évaluation des performances permet de détecter et subir des conditions dégradées de travail, soit en :

- Détectant les cas dégénérés et en les signalant, par exemple, lumière insuffisante dans la pièce qui ne permet pas le bon fonctionnement de la camera et donc la bonne observation.
- Fonctionnant malgré la connaissance de mesures fausses ou de conditions non optimales (les yeux clignent, ne regardent pas à l'endroit demandé, etc.).

Le système est capable de donner des indications sur sa capacité à réussir la mesure ou à la corriger. Il permet d'ajuster la répétition de la mesure à la demande ou en fonction des performances désirées.

### Système proposé et protocoles principaux

Le système proposé suit les recommandations ci-dessus. Ce système a pour objectif d'être facile d'accès, d'un prix abordable et adapté au budget de tout opticien ou e-opticien.

### Composition

Le système utilise :

- une caméra, qui permet l'observation. La technologie permet d'utiliser de simples caméras du marché actuel et d'obtenir des résultats très précis.
- un ordinateur connecté à internet, équipement dont disposent déjà la plupart des opticiens. Leurs logiciels de gestion de stock (PMS) leur demandent souvent d'avoir un tel équipement, afin d'être connecté avec leurs divers fournisseurs et prestataires.
- Une mire, pour certaines formes de notre produit. En fonction du lieu d'utilisation, cette mire peut varier. Pour une utilisation par internet pour les e-opticiens, n'importe quel type d'objet dont la taille est connue peut servir de mire comme une simple carte de fidélité ou de crédit. En magasin, des clips sur les montures pourront être choisis.

### Principe

Le système reconstruit le système mécanique des yeux et mesure le PD3D, en observant le visage et les regards du sujet dans diverses configurations. Le système des yeux en 3D est reconstruit, son rapport à la mire, au visage de l'utilisateur, et au porté des lunettes. A partir de ces informations, la technologie est en mesure de donner les valeurs précises des grandeurs : distance pupillaire PD, monoPD et hauteurs. A partir de ces résultats, il est possible d'ajouter toutes les mesures et protocoles spécifiques pour des activités et particularités de visée (strabisme, etc.), et de qualifier les paramètres de vue plus précisément en regard des nouvelles mesures utiles aujourd'hui pour le montage des verres nouvelle génération.

Les calculs et l'interaction peuvent être répartis sur la machine cliente, et des serveurs de calculs déportés, ce qui rend la technologie robuste aux problématiques d'équipement et au contexte technique local. La mise à jour des versions et l'intégration de nouvelles directives et de nouveaux protocoles est de ce fait instantanée.

Enfin, l'utilisation de la vidéo garantit la capture d'un nombre conséquent d'images qui permet d'effectuer toutes les statistiques nécessaires sur la qualité du protocole et de proposer une mesure fiable.

### Protocoles associés

Le protocole de mesure est court et simple à effectuer. Il existe avec diverses mires, mais suit toujours le principe suivant :

A chaque point apparaissant à l'écran (jusqu'à 3 points par exemple) (en général de 1 à 50, 1 à 20, de préférence 2 à 7), le fixer du regard, et effectuer un mouvement de la tête comme par exemple tourner la tête (dire « non ») lentement.

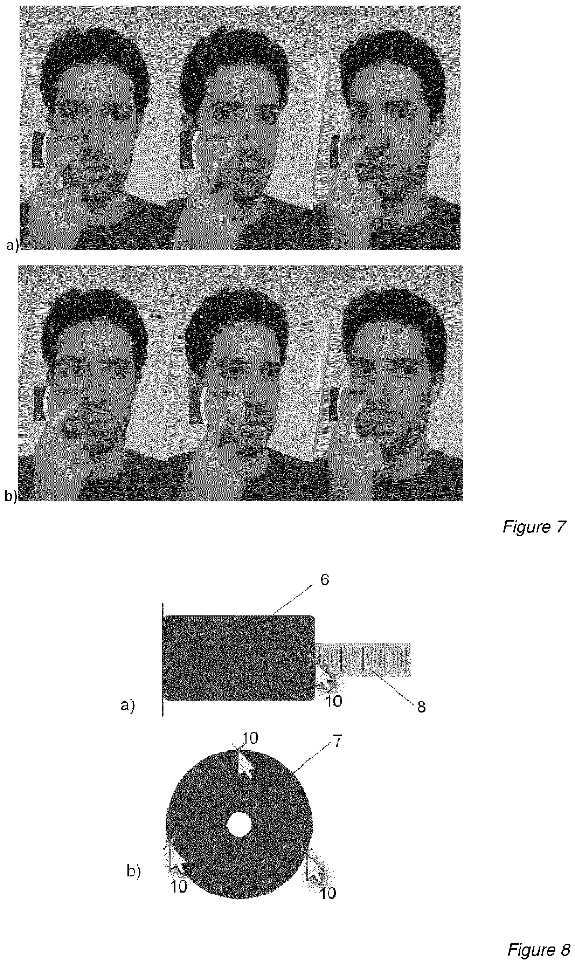

Ce protocole peut s'effectuer dans toutes les conditions en utilisant une mire comme par exemple une carte format « carte de crédit » placée n'importe où sur le visage. La figure 7 montre un exemple des images extraites d'une séquence vidéo.

Le scenario est le suivant :

Si la caméra n'est pas calibrée, on effectue le scénario de calibrage.

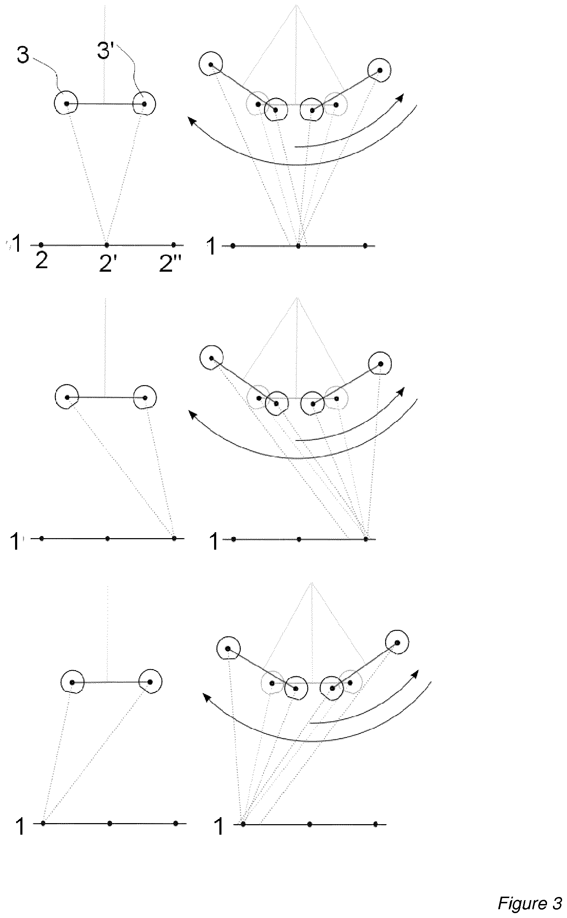

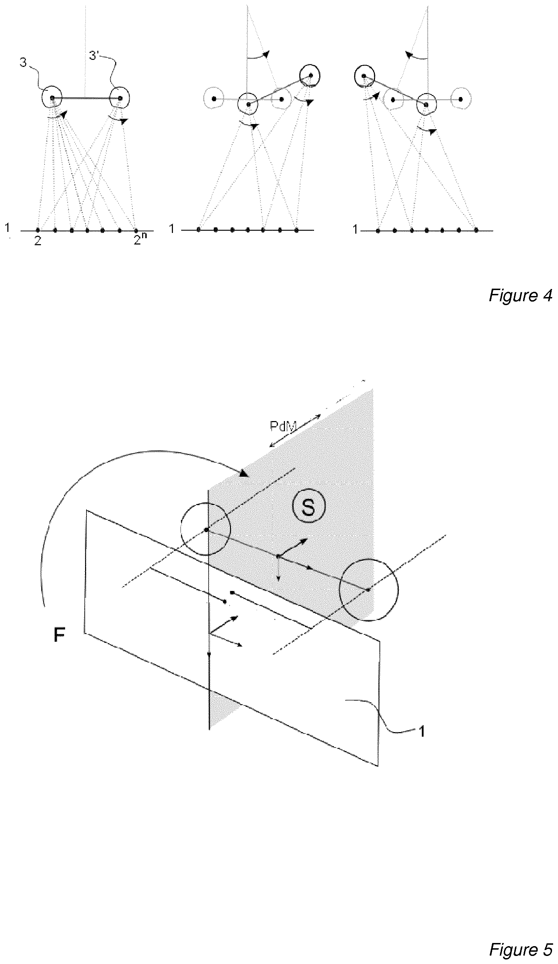

L'utilisateur fixe avec ses yeux (3, 3') des points sur un écran (1), et tourne la tête en regardant ces points (2, 2', 2", $2^n$) un à un. Les figures 3 et 4 illustrent des implémentations de ce scénario.

Afin de reconstruire métriquement le système des yeux en 3D, il est modélisé avec les systèmes suivants

- Un système OEil : P : hdi, dr, rxe,rye,
- Un système Yeux : S, hpd, $T_{SM}$, $R_{SM}$.
- Un système Mire : M, $T_M$, $R_M$.
- Un système caméra : W, K.
- Un système écran : 1,

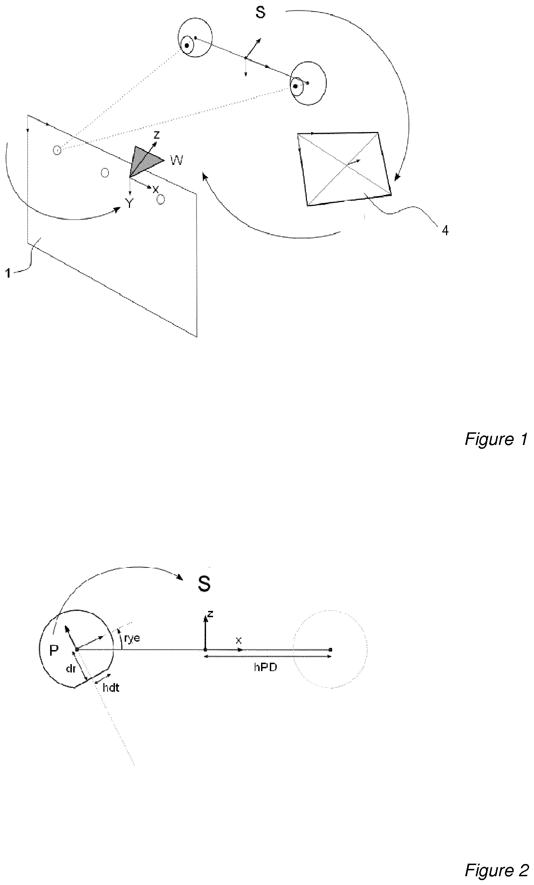

et de la façon suivante (illustré sur les figures 1 et 2):

- le repère caméra W définit l'origine et le repère de l'espace 3D,
- la caméra possède des caractéristiques de calibration K permettant de relier les coordonnées spatiales d'un point de l'espace avec le point associé dans l'image prise par la camera et affiché sur un écran (1),
- un ensemble de points 3D, constituant la mire (4), subissent une translation spatiale $T_M$ et une rotation $R_M$ dans l'espace caméra.

Un premier point $M_0$ de l'ensemble de points 3D de la mire est relié au système des yeux en un point (S) de façon rigide. La transformation rigide reliant la mire (4) au système des yeux est définie par une translation $T_{SM}$ et une rotation $R_{SM}$ dans l'espace centré en $M_0$. Dans l'espace caméra le système des yeux d'origine S subit donc la composition des transformations $(T_{SM}, R_{SM})$ et $(T_M, R_M)$. Le système des yeux est composé de 2 systèmes « oeil » distants de hpd sur l'axe horizontal dans le repère centré en S.

Chaque système « oeil » est modélisé par un centre de rotation P (S est donc le barycentre des points P de chaque oeil), relié à une distance dr à un disque de centre I et de rayon hdi représentant l'iris. Ces disques représentant les iris possèdent 2 degrés de libertés dans le système centré en P : 2 rotations rxe et rye correspondant respectivement au mouvement de rotation de bas en haut de l'iris et au mouvement de rotation de gauche à droite de l'iris dans la cavité oculaire.

La transformation $(T_M, R_M)$ modélise donc la translation et la rotation du mouvement de la tête subit par la mire M solidaire au visage. La transformation $(T_{SM}, R_{SM})$ modélise la relation rigide entre les centres de rotation des globes oculaires et la mire M (tous les 2 solidaires au visage). hpd représente la moitié du « PD3D » recherché et dr représente le rayon longitudinal du globe oculaire. hdi est le rayon de l'iris. Enfin, rxe et rye modélisent les mouvements de rotation des globes oculaires subis par les iris.

Des variantes de modélisations peuvent évidemment être proposées sans gêner la résolution (par exemple modéliser l'œil comme une ellipsoïde au lieu d'un segment et d'un disque).

Dans ce système, seules les dimensions de la mire sont connues avec précision. Ce qui signifie que seule les positions de l'ensemble des points constituants cette mire par rapport au premier point $M_0$ sont connues avec précision.

D'autres grandeurs peuvent être fixées statistiquement comme le rayon moyen de l'iris (hdi) ou le rayon longitudinal moyen du globe oculaire humain (dr). La reconstruction métrique du système des yeux nécessite de retrouver les valeurs inconnues du système que sont dr, hdi, hpd, $T_{SM}$, $R_{SM}$, $T_M$, $R_M$, rxe pour chaque oeil (rxeL et rxeR) et rye pour chaque oeil (ryeL et ryeR).

Après résolution des valeurs inconnues, le système des yeux est métriquement reconstruit selon notre modélisation. Ainsi il est possible de retrouver des mesures 3D indépendantes comme le PD3D d'un utilisateur (distance entre les centres de rotation des yeux), les chemins de visée, ou des mesures 3D fonction du positionnement d'une paire de lunettes par rapport au système des yeux (monoPD, hauteurs, angle pentoscopique ...).

Afin de retrouver les valeurs inconnues du modèle, il est proposé de placer une caméra sur un écran, de placer l'utilisateur à une distance comprise entre 20 et 40 cm environ de la caméra et d'acquérir des images via la caméra pendant que l'utilisateur tourne la tête de gauche à droite en fixant plusieurs points distincts espacés d'une dizaine de centimètres sur l'écran. Ces dimensions garantissent la convergence de la résolution. (L'important étant l'angle de visée). Il est ainsi possible d'obtenir ainsi plusieurs séries d'images dans lesquelles l'utilisateur regarde un point fixe avec des rotations du visage différentes. Le protocole est présenté sur la figure 4.

Deux sortes de valeurs inconnues se distinguent:

- Valeurs inconnues ayant une mesure par image ($T_M$, $R_M$, rxeL, rxeR, ryeL et ryeR).
- Valeurs inconnues ayant une mesure globale (dr, hdi, hpd, $T_{SM}$, $R_{SM}$).

Comme indiqué plus haut $T_{SM}$, et $R_{SM}$ peuvent être calculés par image, si on ne considère pas la position de la mire constante sur la surface au cours de l'expérience (mais solidaire au visage). Pour la suite de la description, nous considérons ces paramètres ayant une valeur constante sur l'ensemble de l'expérience : la mire reste solidaire au visage à la même position et orientation sur sa surface, à de petites variations près..

Le choix de la méthode de résolution du système dépend des algorithmes utilisés pour la détection et l'alignement de la mire, la détection et l'alignement des iris, l'évaluation des performances avec notamment la capacité à détecter les images dans lesquelles la mire n'est plus solidaire du visage.

Dans le contexte où l'on est capable de détecter si la mire n'est plus solidaire du visage, il est possible de résoudre le système en deux étapes :

La première étape consiste à trouver la transformation $(T_M, R_M)$ subie par l'ensemble des points de la mire dans chaque image acquise. Deux méthodes sont possibles:

Méthode 1 :

Pour chaque image il y a minimisation par différence de points (minimisation classiquement résolue par des algorithmes de type Gauss-Newton)

On cherche à minimiser l'expression suivante :

$$argmin_{Rm,Tm} \sum_{i=1}^{nPts} [P_{2D}(i) - Proj(Dp(P_{3D}(i), Rm, Tm)]^2$$

avec

Rm, matrice de rotation 3D
Tm, vecteur translation 3D
nPts, nombre de points projetés
$P_{3D}$, coordonnées 3D de la mire
$P_{2D}$, coordonnées 2D de la mire dans l'image (coins, points contours, points caractéristiques)
Proj, fonction projetant un point 3D dans l'image (modèle sténopé)

$$\text{Proj}(P_{3D}) = \begin{bmatrix} x/z \\ y/z \end{bmatrix}$$

où

$$\begin{bmatrix} x \\ y \\ z \end{bmatrix} = KM * P_{3D}$$

et KM, matrice de calibrage de la camera (précisions dans la partie calibrage)

$$KM = \begin{bmatrix} fx & suv & u0 \\ 0 & fy & v0 \\ 0 & 0 & 1 \end{bmatrix}$$

$D_p$, fonction appliquant la matrice de rotation R et le vecteur de translation T à un point 3D pour un déplacement en 3D

$$D_p(P3D) = R*P3D + T.$$

Méthode 2:

Pour chaque image il y a minimisation par différence de texture selon la formule suivante :

$$argmin_{Rm,Tm} \sum_{i=1}^{nPixels} [Tex(i) - I(W(Proj(Dp(P_{3D}, Rm, Tm), i))]^2$$

avec

Rm, matrice de rotation 3D
Tm, vecteur translation 3D
nPixels, nombre de pixels composant la texture Tex de la mire
I, Image

W, fonction permettant de déformer l'image (I) à partir de coordonnées image 2D vers les coordonnées Texture (Tex)
Proj, fonction projetant un point 3D dans l'image (modèle sténopé)
$D_p$, fonction appliquant la matrice de rotation R et le vecteur de translation T à un point 3D pour un déplacement en 3D.

La deuxième étape consiste à trouver ensuite les valeurs des variables globales $T_{SM}$ $R_{SM}$, hdi , hpd ainsi que les valeurs rxeL, rxeR, ryeL, ryeR pour chaque image acquise. De la même façon, deux méthodes sont possibles :

Méthode 1 :

Sur l'ensemble des images il y a minimisation par différence de points (rxe et rye désignent respectivement à la fois rxeL, rxeR et ryeL, ryeR) selon la formule suivante :

$$\underset{\substack{hpd,hdi,dr,\\rxe,rye,\\Rsm,Tsm}}{\text{argmin}} \sum_{i=1}^{nPts} \sum_{j=1}^{nIm} \left[ \begin{array}{c} P_{2D}(i,j) - \\ Proj(Dp(Dp(P_{3D}(i,hpd,hdi,rxe(j),rye(j)),Rsm,Tsm),Rm(j),Tm(j))) \end{array} \right]^2$$

avec

hpd, distance entre le centre du système des yeux et le centre de rotation de chaque oeil.
hdi, rayon du disque représentant l'iris.
rxe, rye, rotations des iris autour du centre de l'œil (par image).
Rsm, matrice de rotation 3D rigide.
Tsm, vecteur translation 3D rigide.
Rm, matrice de rotation 3D (par image)
Tm, vecteur translation 3D (par image)
nPts, nombre de points projetés
nIm, nombre d'images acquises
$P_{3D}$, coordonnées 3D des contours des iris (échantillonnage sur les contours des disques)
$P_{2D}$, coordonnées 2D des contours des iris dans l'image
Proj, fonction projetant un point 3D dans l'image (modèle sténopé)
$D_p$, fonction appliquant la matrice de rotation R et le vecteur de translation T à un point 3D pour un déplacement en 3D.

Méthode 2: sur l'ensemble des images, minimisation par différence de texture

$$\underset{\substack{hpd,dr,hdi,\\rxe,rye,\\Rsm,Tsm}}{\text{argmin}} \sum_{i=1}^{nPix} \sum_{j=1}^{nIm} \left[ Tex(i) - I(W(Proj(Dp(\begin{array}{c} Dp(P_{3D}(i,hpd,hdi,rxe(j),rye(j)),Rsm,Tsm),\\ Rm(j),Tm(j) \end{array})))) \right]^2$$

avec

hpd, distance entre le centre du système des yeux et le centre de rotation de chaque oeil.
hdi, rayon du disque représentant l'iris.
rxe, rye, rotations des iris autour du centre de l'œil (par image).
I, Image
W, fonction permettant de déformer l'image (I) à partir de coordonnées image 2D vers les coordonnées Texture (Tex)
Rsm, matrice de rotation 3D rigide.
Tsm, vecteur translation 3D rigide.
Rm, matrice de rotation 3D (par image)
Tm, vecteur translation 3D (par image)
nPix, nombre de pixels composant la texture Tex de la mire
nIm, nombre d'images acquises
$P_{3D}$, coordonnées 3D des contours des iris (échantillonnage sur les contours des disques)
$P_{2D}$, coordonnées 2D des contours des iris dans l'image

Proj, fonction projetant un point 3D dans l'image (modèle sténopé)

D$_p$, fonction appliquant la matrice de rotation R et le vecteur de translation T à un point 3D pour un déplacement en 3D.

Dans une première variante de la résolution, dans le cas où la mire n'est pas continument solidaire du visage avec la même transformation (T$_{SM}$, R$_{SM}$), les valeurs inconnues sont distinguées de la façon suivante:

- Valeurs inconnues ayant une mesure par image (T$_M$, R$_M$, T$_{SM}$, R$_{SM}$, rxeL, rxeR, ryeL et ryeR).
- Valeurs inconnues ayant une mesure globale (dr, hdi, hpd).

La résolution peut alors être réalisée par la minimisation de la formule suivante :

$$\underset{\substack{hpd,dr,\\hdi,\\rxe,rye,\\Rsm,Tsm,\\Rm,Tm}}{argmin} \sum_{i=1}^{nPts} \sum_{j=1}^{nIm} \left[ \begin{array}{c} P_{2D}(i,j) - \\ Proj(Dp(Dp\left(P_{3D}\left(i,hpd,hdi,rxe(j),rye(j)\right),Rsm(j),Tsm(j)\right), \\ Rm(j),Tm(j)) \end{array} \right]^2$$

sur l'ensemble des images acquises.

La minimisation est décrite en différence de points mais est aussi réalisable en différence de texture.

La résolution en deux étapes précédemment décrite peut éventuellement être utilisée comme étape d'initialisation.

La modélisation permet de résoudre les différentes minimisations exprimées grâce à des algorithmes classiques de type Gauss-Newton dont les paramètres 2D sont remplacés par les paramètres 3D de la présente invention.

Etant donné qu'il n'y a besoin que d'un nombre limité d'images pour reconstruire métriquement le système des yeux et que l'acquisition des images peut se faire en mode vidéo, il est alors possible de réaliser une validation de performances à l'aide d'autres images de la vidéo. Cela consiste à ajouter plusieurs lots d'images aux images nécessaires à la reconstruction et éventuellement d'enlever certaines images de façon aléatoire, de procéder à la reconstruction et de conforter les résultats obtenus.

Une reconstruction métrique du visage peut être envisagée sans mire solidaire au visage. On propose alors d'enrichir la modélisation exposée par un angle *drye* permettant d'expliquer le fait que la ligne de visée (droite passant par le centre du globe oculaire et le centre de l'iris) ne passe pas par le point fixé à l'écran mais est décalé d'un angle drye sur la rotation gauche-droite de l'œil.

Ainsi, il n'est plus nécessaire de rechercher un angle ryeL et ryeR pour chaque image mais simplement un dryeL et dryeR global : Rye(L ou R) = drye(L ou R) + angle présent entre l'axe horizontal et la droite reliant le centre du globe oculaire et le point fixé à l'écran.

La reconstruction est alors rendue possible par cette simplification du nombre de paramètres et par le fait que la distance entre les points fixés soit connue sur l'écran. La mire 3D solidaire au visage est alors remplacée par les points affichés sur l'écran dont l'espacement est connu. Le protocole est aussi enrichit par des points intermédiaires comme suggéré sur la figure 5.

Enfin, dans toutes les modélisations ici envisagées, la reconstruction du système des yeux peut être éventuellement aidée par la reconstruction de points caractéristiques présents sur le visage. En effet, repérer des points caractéristiques du visage dans les images acquises, peut être considérer comme suivre et reconstruire des points d'une mire 3D dont la position est inconnue mais qui est physiquement solidaire au visage.

*Mise en œuvre*

Afin d'obtenir les indices images qui permettent la résolution décrite, il est nécessaire d'ajouter des traitements qui permettent :

- La détection et alignement de la carte dans toutes les images nécessaires,
- Le calibrage de la camera,
- La détection et l'alignement des iris dans toutes les images nécessaires,
- L'évaluation des performances et le calcul de la stabilité du résultat.

Pour cela, le procédé suivant est utilisé:

1. détection et alignement de la carte

L'utilisateur présente la carte à la caméra.

L'algorithme détecte automatiquement la carte et apprend son apparence.

L'utilisateur met la carte sur son visage, comme le proposent les configurations de la figure 4. Pour chaque image nécessaire au calcul de la mesure, l'apparence est utilisée pour retrouver la carte. Ensuite, un alignement précis est effectué et la pose 3D de la carte est déduite.

Dans le cas où il faut calibrer la caméra, l'utilisateur présente des configurations différentes de la carte à la caméra. La carte est suivie et alignée dans chaque image. Le calibrage est résolu selon la méthode de la section Calibrage. Les étapes de calibrage et protocole de prise de vue PD peuvent être confondues.

2. détection et alignement des yeux

Pour cela, on utilise un modèle d'apprentissage des yeux appris dans les conditions du protocole. Les yeux sont détectés puis alignés dans toutes les images pour lesquelles la carte a été traitée.

3. contrôle et performances

La mesure est calculée, ainsi que la pose 3D de tous les éléments du système et leurs relations. Une caractérisation automatique de la qualité de chaque image et son apport pour le résultat final est effectuée. Parmi les images acquises, on effectue divers tirages d'un certain nombre d'images nécessaire à la reconstruction et résolvons à nouveau le système, ce qui permet d'en déduire la confiance de l'estimation. On évalue aussi par la même si le protocole a été bien réalisé : la carte n'a pas bougé sur le visage pendant la prise de vue, les yeux sont restés suffisamment ouverts, etc.

*Principe d'alignement*

En général, les méthodes de détection et alignement ont deux types d'approches opposées : une approche dite « bottom-up », qui consiste à déduire d'un ensemble de caractéristiques trouvées dans l'image, des structures connues et à en déduire des transformations ou candidats potentiels des paramètres cherchés, et une approche dite « top-down », qui se sert d'une connaissance sur l'apparence pour la rechercher dans l'image. Que cette apparence soit le signal image lui-même ou transformé par des filtres, le support de la structure cherchée est évalué sur l'image pour déterminer la vraisemblance des paramètres candidats.

Le système selon l'invention utilise le principe d'alignement suivant, qui regroupe ces deux approches et en tire le meilleur parti :

Dans un premier temps, on effectue un apprentissage de l'apparence de l'objet cherché. On génère ensuite des apparences et positions probables de cet objet dans l'espace image considéré et on cherche ces représentations, appelées gabarits (ou templates selon la terminologie anglaise) dans l'image. Ces représentations peuvent être paramétrées ou non.

Ensuite, on crée l'image des contours de l'image considéré, par un filtre de Canny par exemple, et on cherche des structures géométriques simples qui correspondent à une description paramétrée des contours des apparences possibles de l'objet recherché. Pour les yeux, il s'agit d'ellipses, pour la carte, il s'agit d'un assemblage de droites et de coins.

On choisit la solution qui a le meilleur support, et considérant la valeur paramétrique des indices images, on est capables de déduire les configurations 3D à l'origine de ces projections selon les équations présentées plus haut. La résolution peut se faire par méthode stochastique robuste, comme un algorithme de RANSAC (Fischler, 1981) et ses dérivés (MLESAC, etc .) ou par alignement de contours.

Cette méthode est robuste aux changements de qualité d'image, d'illumination et d'occultations de l'objet. L'étape d'alignement géométrique est robuste aux erreurs possibles d'échelle, de position ou d'orientation 2D pouvant être commises par les techniques par apparence.

Cette méthode peut inclure les méthodes habituelles "top-down" ou "bottom-up" à l'intérieur du gabarit considéré pour rendre le suivi plus robuste. Par exemple, des points d'intérêt de type Harris et des descripteurs de type SIFT (de l'anglais "Scale-invariant feature transform") ou SURF (de l'anglais "Speeded Up Robust Feature") peuvent être utilisés pour permettre un suivi des attributs du template (Mikolajcziyk, 2005). On suit une approche similaire à celle de (Hinterstoisser, 2009), à la différence que les gabarits et éléments de texture sont organisés et contrôlés, et que l'alignement est géométrique et non par apparence.

On englobe dans cette méthode son utilisation en multirésolution ainsi que le découpage en sous-structures du template, comme par exemple les points d'intérêt cités plus haut ou le découpage en sous-templates organisées spatialement.

*Détection de carte, suivi et alignement*

Pour la première détection, on demande à l'utilisateur de placer la carte dans un gabarit présenté à l'écran. On utilise alors l'algorithme d'alignement.

On apprend ensuite la texture en effectuant une transformation des points de la carte de l'image d'apprentissage telle que la forme de la texture suivent les ratios de la carte, en maximisant la surface couverte et en minimisant la distorsion par rapport à l'image d'apprentissage. On cherche pour cela l'homographie qui permet de passer des points trouvés à un rectangle de taille max (hauteur, largeur parmi les 4 points considérés), avec max (a,b) fonction maximum entre deux réels. On interpole la texture acquise selon des méthodes connues pour remplir cette texture de référence notée *CBTex* dans la suite de la description.

Pour le suivi et les détections ultérieures, on cherche la texture de la carte dans l'image en générant un ensemble de gabarit (la texture carte subit alors une transformation 2D projective) relatifs à des poses 3D possibles de cartes.

La déformation de la texture 2D s'exprime de la manière suivante : si R,t est la position de la carte dans la scène, alors l'homographie associée s'exprime H = [R( :,1 :2) t], avec R( :,1 :2) les deux premières colonnes de la matrice de R. Un autre moyen de calculer l'homographie est de calculer les projections des coins de la carte sur l'image considérée et de déduire l'homographie entre les points de la texture et ceux de l'image de projection pour les paramètres de scène 3D considérés.

La texture CBTex est ainsi déformée pour chaque gabarit créé. Pour chaque gabarit, une recherche dans l'image est effectuée et des scores de corrélation sont établis. De nombreuses méthodes connues de l'homme du métier existent pour réaliser une telle correspondance de texture (en anglais « template matching »). On peut citer la corrélation connue sous le nom ZNCC (pour Zero mean Normalized Cross-Correlation selon la terminologie anglaise) ou l'alignement de T. Kanade (Baker, 2004), ou ESM (Benhimane, 2007).

On sélectionne le gabarit ayant le meilleur score de corrélation ou d'alignement parmi tous les gabarits produits et testés sur l'image. Comme le gabarit est directement associé à une pose 3D, on déduit les points de la carte et on effectue un algorithme d'alignement géométrique si nécessaire.

Pour les points trouvés, on cherche la meilleure pose qui explique ces points selon la formule suivante :

$$argmin_{Pose} \sum_{i=1}^{nPts} [P_{2D}(i) - Proj(Dp(P_{3D}(i), Pose)]^2$$

*Alignement géométrique de la carte*

Afin de trouver les coins de la carte précisément sans connaître son apparence, on utilise ses caractéristiques géométriques : on décrit la carte comme une série de courbes et droites paramétrées. Cela est valable pour n'importe quel objet mire : lunettes, CD, carte, visage.

On utilise l'image des contours, en appliquant un filtre de Canny à l'image originale. On cherche les ensembles de quatre droites qui peuvent décrire la carte. Afin de restreindre les possibilités, on utilise une solution initiale : un gabarit lorsque la carte est inconnue, la solution du suivi par apparence lorsque la carte est connue, c'est-à-dire les quatre points associés aux coins de la carte.

Pour trouver chaque droite, on utilise un algorithme stochastique (RANSAC (Bolles, 1981), par exemple), en considérant les points contours, et on cherche à estimer une droite. Les intersections des droites trouvées deux à deux donnent les quatre points coins de la carte.

*Détection des yeux, suivi et alignement*

Pour la détection des yeux, on apprend un modèle statistique d'apparence de type connu sous l'acronyme AAM (de l'anglais "Active Appearance Model"), afin de posséder une image de texture des yeux ainsi que leur variabilité d'apparence.

On génère des gabarits probables en fonctions de poses 3D probables pour les images considérées relativement au protocole effectué. Il est possible d'ajouter la connaissance de la pose de carte trouvée précédemment et d'affiner l'échelle des poses probables pour l'image considérée en fonction de la pose de carte.

Les gabarits sont dans un premier temps les images moyennes des modèles d'apparence appris. On aligne ces gabarits moyens par corrélation ZNCC, puis par algorithme d'alignement (Inverse Compositional Algorithm par exemple (Baker, 2004)) en permettant à tout le modèle de forme et d'apparence de varier.

Ce processus est répété pour l'ensemble des images considérées et suivi d'un alignement.

*Alignement géométrique des yeux*

Une fois la position et l'échelle initiales du gabarit donné par l'alignement précédent, on utilise l'image des contours et on cherche des ellipses, supports image des iris, dans la zone considérée. La encore, un algorithme stochastique de tirage de points et d'évaluation du support 2D de la structure ellipse comme RANSAC est utilisé. Cela permet de trouver l'indice image malgré les problèmes nombreux de reflets sur la cornée, ou de clignement des yeux, qui impliquent un changement fort et imprévu d'apparence.

## Mires, calibrage et métriques

### Calibrage de la caméra

Le calibrage consiste à retrouver les caractéristiques intrinsèques de la caméra selon la matrice suivante :

$$\begin{bmatrix} fx & suv & u0 \\ 0 & fy & v0 \\ 0 & 0 & 1 \end{bmatrix}$$

avec :

*fx* et *fy,* les distances focales en largeur et hauteur de pixels (fx et fy peuvent éventuellement être considérées égales à une valeur f),
*(u0, v0),* les coordonnées du centre optique projeté dans l'image (u0 et v0 peuvent être éventuellement être considérés au centre de l'image),
et *suv* un facteur exprimant la non orthogonalité de la camera (généralement considéré comme nul).

Le calibrage permet de relier les coordonnées spatiales d'un point de l'espace avec le point associé dans l'image prise par la caméra. Il est réalisé partir d'images d'une mire 3D dans des rotations et positions différentes. Le calibrage est classiquement résolu à l'aide de mires non planes ou planes fortement texturées.

On propose 2 approches.

Approche 3D 1 : pour l'ensemble des images acquises, minimisation par différence sur des points (résolution classique grâce à des algorithmes de moindres carrés de type Gauss-Newton)

$$argmin_{K,Rm,Tm} \sum_{i=1}^{nPts} \sum_{j=1}^{nIm} [P_{2D}(i,j) - Proj(Dp(P_{3D}(i), Rm(j), Tm(j), K)]^2$$

avec

K, le vecteur regroupant les caractéristiques de la camera (focale f, coordonnées u0, v0 du centre optique dans l'image)
Rm, matrice de rotation 3D de la mire (par image)
Tm, vecteur translation 3D de la mire (par image)
nPts, nombre de points projetés
nIm, nombre d'images acquises
$P_{3D}$, coordonnées 3D de la mire
$P_{2D}$, coordonnées 2D de la mire dans l'image (coins, points contours, points caractéristiques)
Proj, fonction projetant un point 3D dans l'image (modèle sténopé)

$$\mathrm{Proj}(P_{3D}) = \begin{bmatrix} x/z \\ y/z \end{bmatrix}$$

où

$$\begin{bmatrix} x \\ y \\ z \end{bmatrix} = KM * P_{3D}$$

et KM est la matrice de calibrage de la caméra

$$KM = \begin{bmatrix} fx & suv & u0 \\ 0 & fy & v0 \\ 0 & 0 & 1 \end{bmatrix}$$

$D_p$, fonction appliquant la matrice de rotation R et le vecteur de translation T à un point 3D pour un déplacement en 3D

$$D_p(P3D) = R*P3D + T.$$

Approche 3D 2 : pour l'ensemble des images acquises, minimisation par différence sur des textures

$$argmin_{Rm,Tm,K} \sum_{i=1}^{nPix} \sum_{j=1}^{nIm} [Tex(i,j) - I(W(Proj(Dp(P_{3D}, Rm(j), Tm(j), K), i)))]^2$$

avec

Rm, matrice de rotation 3D (par image)
Tm, vecteur translation 3D (par image)
nPix, nombre de pixels composant la texture Tex de la mire
I, Image
W, fonction permettant de déformer l'image (I) à partir de coordonnées image 2D vers les coordonnées Texture (Tex)
Proj, fonction projetant un point 3D dans l'image (modèle sténopé)
$D_p$, fonction appliquant la matrice de rotation R et le vecteur de translation T à un point 3D pour un déplacement en 3D.

La modélisation permet de résoudre ces différentes minimisations grâce à des méthodes de type Gauss-Newton classiques dont les paramètres 2D sont remplacés par nos paramètres 3D.
Une initialisation de K peut être obtenue grâce aux homographies reliant dans chaque image les coordonnées métriques du plan 3D de la mire aux coordonnées image (Maybank, 1999) et (Zhang, 2000).

## *Types de mire*

Tout objet de taille initialement connue et qui peut se trouver solidaire au visage peut servir de mire comme par exemple une carte bancaire, une carte de fidélité au format carte bancaire, un CD (compact-disc), une paire de lunettes dont les dimensions sont connues, ou encore un objet connu tenu de façon rigide à une paire de lunettes.
Une modélisation de la reconstruction du système des yeux autorise même de pouvoir se passer d'une mire solidaire au visage. Dans ce cas précis, la mire en question est constituée des points affichés sur l'écran dont la distance entre les points est connue.
Enfin, le visage peut faire office de mire si on connaît ses dimensions 3D. Ces dimensions peuvent être reconstruites grâce à la carte et à la reconstruction de points du visage, ou l'utilisation de modèles statistiques de visage (Vetter, 2005), la métrique étant apportée par notre méthode de résolution et la mire initiale considérée.

## *Calibrage géométrique de l'écran*

Afin de garantir la distance d'écartement des points visés par l'utilisateur, et de l'adapter le cas échéant, il est nécessaire de mesurer son écran dans un espace métrique. Le système peut alors proposer des points de visée adéquats avec la taille de l'écran et ainsi adapter la distance de l'utilisateur pour la bonne réussite du protocole.
Dans le cas ou la mire est un objet plan, tel qu'un Compact Disc ou un carte de crédit, le système lui demande de placer l'objet conte l'écran. Il affiche alors une grille, et l'utilisateur clique sur les bords de l'objet. La résolution de l'écran native étant connue par le programmevia le système de gestion de l'affichage, un simple facteur d'échelle est appliquer pour trouver les dimensions réelles de la dalle, de manière imparfaite mais assez précise pour positionner les points de visée à moins de 5mm d'erreur. La figure 8 donne un exemple de gabarit grille pour une carte de crédit(6) a) ou un CD (7) b), et les points (10) des clics minimaux à effectuer par l'utilisateur. La référence 8 représente un élément de mesure de l'espace métrique.

## Mesures PV en contexte monocaméra

### *Définitions des mesures PV 3D*

Pour les mesures PV, il existe plusieurs manières de les redéfinir, et le système est capable de toutes les mesurer.

On donne des définitions sur les concepts les plus probables issus des définitions classiques, qui sont considérées dans un cadre 2D, dans le sens où les mesures proposées doivent être réalisées dans une position donnée et observée avec une projection orthographique.

On considère un recalage initial selon une rotation rx autour de l'axe x du repère Yeux (S) par rapport à une photo telle que la personne est en position naturelle de port de tête et de lunettes. La définition en 3D peut devenir indépendante du regard, et peut permettre d'adapter le taillage des verres en fonction de chaque distance que l'on voudrait explicitement considérer, car l'expression 3D de ces notions permet de calculer les points d'intersection du regard supposé avec le plan du verre monté.

Pour permettre les mesures 3D PV, on considère que la monture est placée sur le visage, et que la transformation entre les repères montures (F) et système des yeux (S) est calculée. On peut s'en affranchir ou la considérer dans le calcul en fonction du montage des verres désiré.

Pour les monoPD 3D gauche et droit, on considère deux définitions. La première est de mesurer la distance orthogonale entre le plan de symétrie vertical de la monture (PdM) (plan yz dans le repère monture) et la position du centre P de chaque globe oculaire, comme illustré sur les figures. Une deuxième mesure possible est de calculer les points d'intersection entre la ligne de visée 3D de chaque oeil et le plan du verre, pour un regard considéré (en général à l'infini, mais notre proposition 3D permet de déterminer plus précisément chaque regard considéré si nécessaire). La mesure cherchée est alors la distance entre le plan vertical et les points d'intersection.

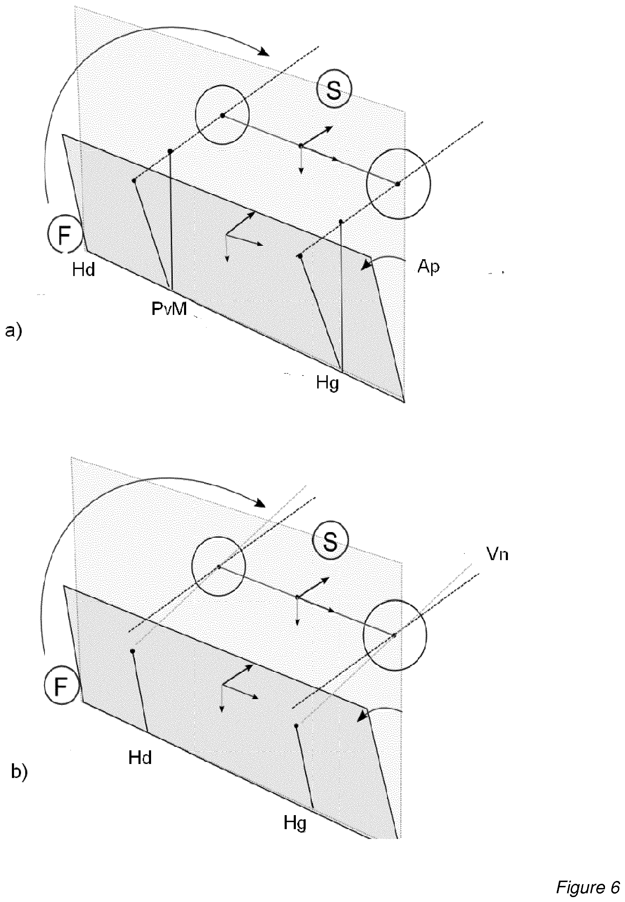

Pour les hauteurs (hauteurs droite ;HD, hauteurs gauches :Hg), on mesure la distance orthogonale entre le plan horizontal passant par l'œil et la droite passant par le point bas de la monture au niveau du verre (PvM), comme illustré sur la figure 6. Cette droite se situe au point bas de la dragée interne. Deux mesures sont alors possibles : distance dans le plan du verre, ou distance dans le plan vertical du repère lunette perpendiculaire au plan horizontal oeil, puisque les repères oeil et lunettes ont été préalablement recalés.

Une variante consiste, comme pour les monoPD (mPD), à calculer les points d'intersection entre les rayons de visée (Vn) et les plans de la lunette que l'on vient de décrire, comme indiqué dans le bas de la figure 6. Le calcul de la distance dans le plan vertical correspond à la représentation 3D de la définition usuelle des hauteurs.

Angle pentoscopique (Ap): par rapport à position naturelle du visage considérée dans nos définitions, l'angle pentoscopique mesure l'angle entre le plan d'inclinaison des verres et le repère recalé du visage en position naturelle.

### *Mesures avec port de montures réelles*

Les mesures PV nécessitent le port de la monture. On les déduit en 3D en repérant les points et lignes 3D permettant leurs mesures. Si la monture est inconnue du système (pas de modèle 3D disponible), on reconstruit les points et courbes 3D nécessaires. On repère pour cela les indices images et les alignons par la méthode alignement texture puis géométrique proposée section principe d'alignement. Si la monture est connue, elle peut constituer une mire. Elle est soit fabriquée explicitement pour la mesure ou c'est une lunette dont on possédé le modèle 3D par design manuel ou reconstruction automatique.

### *Monture inconnue*

Pour une monture inconnue, on prend des images de la monture portée par l'utilisateur. La résolution du PD ayant été effectuée, on retrouve les plans et droites 3D par leurs projections dans les images. Pour le plan de symétrie du monoPD, on recherche la surface support perpendiculaire à ce plan telle que la projection dans les images permette la symétrie de l'apparence de la lunette dans l'espace de la surface support, comme le montrent les équations qui suivent :

$$argmin_{\gamma, Rp, Tp} \sum_{i=1}^{nPix} f_S(i, I(W(\gamma, Rp, Tp)))^2$$

avec

Rp, matrice de rotation 3D du plan de support

Tp, vecteur translation 3D du plan de support

nPix, nombre de pixels composant une moitié de la texture de la paire de lunettes (sans la partie verres)

I, Image

W, fonction permettant de déformer l'image (I) à partir d'un support de courbure $\gamma$, et de pose $(R_p, T_p)$.

$f_S$ , fonction de symétrie : $f_S(i, Image) = Image(i) - Image(i\_symétrique)$.

Pour la droite 3D passant par le bas de la monture, elle est calculée à partir de plusieurs images par l'intersection des plans passant par le centre optique et les droites 2D repérées dans les images, qui peuvent être déterminées par des clics de l'utilisateur ou par une recherche texture et alignement sur une base de connaissance de lunettes apprises (détection et alignement de modèles statistiques).

Pour l'inclinaison du plan des verres, il suffit de trouver la droite de la dragée haute des montures comme indiqué plus haut, et de calculer l'angle entre le repère des yeux et l'inclinaison du plan qui contient ces deux droites 3D.

Pour mieux calculer les points d'intersection du regard et du verre (si on choisit les définitions associées), on peut introduire la connaissance a priori de la forme de la surface du verre.

### Monture de modèle connu

Pour un modèle 3D connu, les points nécessaires sont déjà référencés sur le modèle. Cela peut être fait par méthode manuelle ou automatique, selon des méthodes d'analyse 3D de surface et d'apparence.

Le problème qui se pose est alors de chercher les paramètres de pose du modèle 3D dans la scène, telles que les erreurs de reprojection de la forme de la monture soient minimisées dans les images considérées. On peut utiliser une solution géométrique ou s'aider de l'apparence si celle-ci est disponible. Des techniques classiques de recalage comme (Vetter, 2005) peuvent être utilisées, en prenant pour solution initiale la pose du système des yeux S translatée de - 12mm selon z, ce chiffre étant la distance moyenne verre-œil attendue lors d'un port de lunettes.

### Mesures avec port de montures virtuelles

Si on possède une reconstruction fine du visage 3D de l'utilisateur, alors le positionnement se fait par simulation du port de la lunette sur le visage. On propose un ajustement manuel 3D qui suit les contraintes physiques du visage et de la monture.

Si on ne possède pas un modèle 3D suffisamment précis, un opérateur ou l'utilisateur lui-même ajuste la paire en 3D dans une interface de visualisation. On décrit ici l'ajustement virtuel et son interface :

L'outil montre plusieurs vues de l'utilisateur acquises et qui permettent une bonne visualisation de l'ajustement, ainsi qu'une vue temps réel du flux vidéo. L'utilisateur manipule une vue de contrôle 3D qui permet de déplacer la paire selon des degrés de liberté autorisés : ouverture de branches, rotation, etc. Si le visage 3D est connu, ces mouvements sont contraints par les points de contact des surfaces 3D du visage et des lunettes.

Lorsque la paire est manipulée en 3D, sa projection est effectuée sur l'ensemble des vues réelles statiques et dynamiques en réalité augmentée. L'utilisateur peut ainsi proposer différents ports de tête naturels pour différentes activités et ajuster ses lunettes en fonction. Les mesures 3D nécessaires au montage sont alors enregistrées et analysées par un professionnel.

Cet outil est utilisé par l'utilisateur final pour la prise de vue et le port de tête, et par le professionnel de l'optique pour le choix des vues et mesures pour le réglage des montures et le montage des verres. Ces deux utilisateurs peuvent être présents tous deux au moment de l'essayage virtuel ou le procédé peut être accompli en deux temps ou endroits si l'opticien n'est pas sur le même lieu physique ou dans le même temps de prise de vue. En effet, les images acquises peuvent être rejouées et réajustées par le professionnel plus tard ou depuis un autre lieu en temps réel. Cela permet le traitement déporté ou la prise de mesure en ligne assistée par un professionnel.

Cet outil permet également à l'utilisateur de définir son besoin en verre sans l'inconvénient de l'essayage.

**Autres caractéristique du système**

*Architecture*

Le procédé utilise une architecture Client-serveur qui permet de déporter les calculs, les vidéos et images acquises et répartir les interfaces d'ajustement pour les différents utilisateurs.

*Analyse du comportement oculaire*

Le procédé permet de reconstruire le système oculaire et de trouver pour chaque image acquise les visées effectuées. Connaissant les distances métriques entre les points visés dans le système écran E, on peut définir un modèle de convergence pour toutes les positions acquises et interpoler ce modèle pour d'autres points visés.
On propose ainsi un calibrage du système oculaire en présentant à l'utilisateur une matrice de points qui couvre son écran. Lors d'un suivi de regard, on est en mesure de déduire quels points à l'écran sont regardés par l'utilisateur en temps-réel.
Le procédé décrit fonctionne aussi bien et se simplifie, dès lors qu'on dispose de deux caméras au lieu d'une seule reliées par une contrainte rigide connue ou inconnue. De même, l'utilisation de dispositifs donnant la profondeur est compatible avec les équations et le système décrits dans la présente invention et rajoutent à la stabilité ou immédiateté du résultat.
Le système selon l'invention peut être utilisé pour différentes applications, dont les principales sont les suivantes :

- Mesures SV en ligne : Une carte de fidélité ou un CD peuvent être utilisés pour réaliser le protocole. Certains supports avec caméras intégrées aujourd'hui très répandus facilitent la réalisation.
- Mesures SV Magasin : La mire peut être une carte manufacturée spécialement, un CD, mais aussi des mires qui s'accrochent sur les montures réelles. Dans certains cas, la monture réelle choisie devient la mire et les clips ne sont pas nécessaires.
- Mesures PV Magasin : Les mesures traditionnelles PV devant s'effectuer en portant la monture pour un regard et une position de visage déterminés, la monture est nécessaire. La solution mire-monture répond au besoin de manière automatique. L'utilisation d'une mire plus simple, comme la carte, est possible, en mode semi-assisté par l'opticien : ce dernier donne alors au système quelques indices sur des images. Cette opération est évaluée en temps réel par le système de contrôle des performances.
- Mesures PV en ligne : La monture n'étant pas disponible en ligne pour les mesures PV, la technologie d'essayage virtuel intervient dans le processus de mesure. Elle permet de simuler le porté de la monture et de prendre ainsi les mesures PV attendues. Comme en conditions de porté réel (et même avec plus de confort car l'utilisateur peut se voir en gardant ses propres lunettes lors de l'essayage virtuel), l'utilisateur s'observe dans l'écran (miroir), et peut se voir de plusieurs points de vue. Les mesures PV sont effectuées en 3D et pour autant de configurations de visées que désiré. L'e-opticien peut éventuellement assister et contrôler cette prise de mesure.

**Exemples :**

*Exemple de mesures de PD :*

| Mesure PD | FittingBox Perfect Fit | Pupillomètre |
|---|---|---|
| **Précision** | +-0.2 mm | +-0.25 mm |
| **Stabilité** | +-1 mm | +-1 mm |
| **Répétabilité** | 1 mesure (5 vérifications unitaires intégrées) | 3 à 5 mesures nécessaires. |
| **Précision finale en conditions réelles** | +-0.2mm | +-1mm |

*Exemple de mesure de PV :*

18

|  | monoPD | Hauteurs |
|---|---|---|
| **Précision mesure PV magasin** | +-0.75 mm | +-0.75 mm |
| **Précision mesure PV online** | +- 1mm | +-1 mm |

**Revendications**

1. Procédé de détermination des mesures oculaires et optiques pour la fabrication et le montage de verre de lunettes correcteurs pour un utilisateur, à l'aide d'un ordinateur, d'une mire et d'une caméra calibrée, ces mesures incluant l'une au moins des mesures suivantes : distance pupillaire, distance monopupillaire, hauteurs, angle pentoscopique,

   utilisant un protocole de reconstruction du système des yeux de l'utilisateur en trois dimensions par modélisation du système de l'œil **caractérisé en ce que** la mire est constituée d'un objet de dimensions connues et choisi dans une liste d'objets standards comprenant une carte bancaire, une carte de fidélité au format carte bancaire, un compact-disc, une paire de lunettes, la mire étant maintenu solidaire au visage sans être attachée à une paire de lunettes,
   le procédé comprenant :

   - une étape de modélisation de la translation et de la rotation du mouvement de la tête de l'utilisateur par le calcul de la transformation géométrique comprenant une translation 3D ($T_M$) et une rotation 3D ($R_M$) subie par l'ensemble des points de la mire dans chaque image acquise,
   - une étape pouvant être intégrée à la première consistant à trouver les valeurs des variables globales comprenant une translation 3D rigide ($T_{SM}$), une rotation 3D rigide ($R_{SM}$), un rayon (hdi) du disque représentant l'iris et une distance (hpd) entre le centre du système des yeux et le centre de rotation de chaque oeil, ainsi que les valeurs de rotation des iris autour des centres des yeux pour chaque image acquise
   - une étape de détermination d'au moins une mesure oculaire ou optique dans le système des yeux reconstruits en trois dimensions.

2. Procédé selon la revendication 1, dans lequel le protocole comprend au moins une étape consistant à effectuer les mesures oculaires et optiques pour différents points de visée et différentes orientations de visage de l'utilisateur.

3. Procédé selon la revendication 2, dans lequel les points de visée sont au nombre de trois et les orientations de visage de l'utilisateur sont au nombre de trois.

4. Procédé selon une des revendications 1 à 3, dans lequel la modélisation est réalisée à partir des grandeurs réelles de l'œil qui sont : la taille de l'iris, la taille des globes oculaires, l'orientation des globes oculaires dans leur cavité, et la pose de l'ensemble des deux yeux dans un repère de la caméra.

5. Procédé selon l'une des revendications 1 à 4, dans lequel la mire à la forme d'une carte rectangulaire.

6. Procédé selon une des revendications 1 à 5, dans lequel la mire est contenue dans un écran de visualisation.

7. Procédé selon la revendication 6, dans lequel la détermination de la taille métrique de l'écran se fait grâce à un objet plan.

8. Procédé selon une des revendications 1 à 7, dans lequel la mire est utilisée au début du protocole.

9. Procédé selon une des revendications 1 à 8, comprenant une étape de mesures PV avec un essayage virtuel de lunettes trois dimensions.

10. Procédé selon une des revendications 1 à 9, comprenant une étape d'évaluation de la stabilité et des performances, ce qui permet de détecter les défauts de réalisation et de les corriger.

**Patentansprüche**

1. Verfahren zur Bestimmung okularer und optischer Messungen für die Herstellung und die Montage von Korrekturbrillenglas für einen Benutzer mit Hilfe eines Rechners, eines Testbilds und einer kalibrierten Kamera, wobei diese Messungen mindesten eine der folgenden Messungen einschließen: Pupillenabstand, Monopupillenabstand, Höhen, pentoskopischer Winkel bei Verwendung eines Rekonstruktionsprotokolls des Augensystems des Benutzer in drei Dimensionen durch Modellierung des Augensystems, **dadurch gekennzeichnet, dass** das Testbild aus einem Objekt mit bekannten Abmessungen besteht und aus einer Liste von Standardobjekten ausgewählt ist, umfassend eine Kreditkarte, eine Treuekarte im Format einer Kreditkarte, eine Compact-Disk, eine Brille, wobei das Testbild fest am Gesicht gehalten wird, ohne an einer Brille angebracht zu sein, wobei das Verfahren umfasst:

   - einen Schritt der Modellierung der Translation und der Rotation der Kopfbewegung des Benutzers durch Berechnung der geometrischen Transformation, umfassend eine 3D-Translation ($T_M$) und eine 3D-Rotation ($R_M$) aller Punkte des Testbilds in jedem erfassten Bild,
   - einen Schritt, der in den ersten integrierbar ist, der darin besteht, die Werte der globalen Variablen zu finden, umfassend eine feste 3D-Translation ($T_{SM}$), eine feste 3D-Rotation ($R_{SM}$), einen Radius (hdi) der Scheibe, die die Iris darstellt, und einen Abstand (hpd) zwischen dem Zentrum des Augensystems und dem Rotationszentrum jedes Auges sowie die Rotationswerte der Iriden um Zentren der Augen für jedes erfasste Bild,
   - einen Schritt des Bestimmens mindestens einer okularen oder optischen Messung im in drei Dimensionen rekonstruierten Augensystem.

2. Verfahren nach Anspruch 1, wobei das Protokoll mindestens einen Schritt umfasst, der darin besteht, die okularen und optischen Messungen für verschiedene Zielpunkte und verschiedene Ausrichtungen des Gesichts des Benutzers durchzuführen.

3. Verfahren nach Anspruch 2, wobei die Zielpunkte drei sind und die Ausrichtungen des Gesichts des Benutzers drei sind.

4. Verfahren nach einem der Ansprüche 1 bis 3, wobei die Modellierung ausgehend von den realen Größen des Auges durchgeführt wird, die sind: die Größe der Iris, die Größe der Augäpfel, die Ausrichtung der Augäpfel in ihrer Höhle und die Stellung der beiden Augen in einem Bezugssystem der Kamera.

5. Verfahren nach einem der Ansprüche 1 bis 4, wobei die Zielscheibe die Form einer rechteckigen Karte hat.

6. Verfahren nach einem der Ansprüche 1 bis 5, wobei die Zielscheibe in einem Visualisierungsbildschirm enthalten ist.

7. Verfahren nach Anspruch 6, wobei die Bestimmung der metrischen Größe des Bildschirms anhand eines ebenen Objekts erfolgt.

8. Verfahren nach einem der Ansprüche 1 bis 7, wobei die Zielscheibe zu Beginn des Protokolls verwendet wird.

9. Verfahren nach einem der Ansprüche 1 bis 8, umfassend einen PV-Messschritt mit einer dreidimensionalen virtuellen Anprobe der Brille.

10. Verfahren nach einem der Ansprüche 1 bis 9, umfassend einen Schritt der Beurteilung der Stabilität und der Leistungsfähigkeit, der erlaubt, Fehler bei der Realisierung zu ermitteln und sie zu korrigieren.

**Claims**

1. A method for determining ocular and optical measurements for the fabrication and mounting of lenses of corrective spectacles for a user, assisted by a computer, a test object and a calibrated camera, these measurements including at least one of the following measurements: pupillary distance, monopupillary distance, heights, pantoscopic angle, using a protocol of three-dimensional reconstruction of the eye system of the user by modelling the eye system, **characterized in that** the test object consists of an object with known dimensions and selected from a list of standardized objects comprising a bank card, a loyalty card with a bank card format, a compact disc, a pair of spectacles, the test object being held secured to the face without being attached to a pair of spectacles, the method comprising:

- a step of modelling the translation and the rotation of the movement of the head of the user through the calculation of the geometric transformation comprising a 3D translation ($T_M$) and a 3D rotation ($R_M$) undergone by all of the points of the test object in each acquired image,

- a step which may be integrated to the first one consisting in finding the values of the global variables comprising a rigid 3D translation ($T_{SM}$), a rigid 3D rotation ($R_{SM}$), a radius (hdi) of the disc representing the iris and a distance (hpd) between the centre of the eye system and the centre of rotation of each eye, as well as the values of the rotation of the irises about the centres of the eyes for each acquired image

- a step of determining at least one ocular or optical measurement in the eye system reconstructed in three dimensions.

2. The method according to claim 1, wherein the protocol comprises at least one step consisting in carrying out the ocular and optical measurements for various points of sight and various orientations of the face of the user.

3. The method according to claim 2, wherein the points of sight are three in number and the orientations of the face of the user are three in number.

4. The method according to one of claims 1 to 3, wherein the modelling is performed based on real quantities of the eye which are: the size of the iris, the size of the eyeballs, the orientation of the eyeballs in their socket, and the pose of the set of two eyes in a reference frame of the camera.

5. The method according to one of claims 1 to 4, wherein the test object has the shape of a rectangular card.

6. The method according to one of claims 1 to 5, wherein the test object is contained in a visualization screen.

7. The method according to claim 6, wherein the determination of the metric size of the screen is done using a planar object.

8. The method according to one of claims 1 to 7, wherein the test object is used at the start of the protocol.

9. The method according to one of claims 1 to 8, comprising a step of PV measurements with a virtual try-on of three-dimensional spectacles.

10. The method according to one of claims 1 to 9, comprising a step of evaluating the stability and performances, which makes it possible to detect production defects and correct them.

*Figure 1*

*Figure 2*

Figure 3

Figure 4

Figure 5

*Figure 6*

a)

b)

*Figure 7*

a)

b)

*Figure 8*

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2914173

- FR 2915290

**Littérature non-brevet citée dans la description**

- **ZEISS.** *Optometry product Guide*

- Ophthalmic lenses and dispensing. *Butterworth-Heinemann Optician,* 1999, ISBN 0750641584